Europäisches Patentamt
European Patent Office
Office européen des brevets

(19) (11) Publication number: 0 293 555 A2

# (12) EUROPEAN PATENT APPLICATION

(21) Application number: 88102764.3

(22) Date of filing: 24.02.88

(51) Int. Cl.[4]: C08G 65/32 , C08G 65/24 , C06B 45/10

(30) Priority: 01.06.87 US 56026

(43) Date of publication of application: 07.12.88 Bulletin 88/49

(84) Designated Contracting States: BE CH DE FR GB IT LI NL SE

(71) Applicant: Rockwell International Corporation
2230 East Imperial Highway
El Segundo, California 90245(US)

(72) Inventor: Frankel, Milton Bernard
5008 Veloz
Tarzana California 91356(US)
Inventor: Wilson, Edgar Roland
7888 Lilac Lane
Simi Valley California 93063(US)

(74) Representative: Wächtershäuser, Günter, Dr.
Tal 29
D-8000 München 2(DE)

## (54) Azide-terminated azido compound.

(57) An energetic azide-terminated azido compound and a method for producing same is disclosed.

EP 0 293 555 A2

# AZIDE-TERMINATED AZIDO COMPOUND

## Background of the Invention

### 1. Field of the Invention

This invention relates to an energetic azide-terminated azido plasticizer, and is particularly directed to a method for producing this compound.

### 2. Description of Prior Art

Commercial polyalkaline ether glycols (1) have been nitrated to give the corresponding polyalkaline ether glycol dinitrates (2):

$$\underset{\underline{1}}{HO\text{-}(CH_2CH_2O)_n\ CH_2CH_2OH} \xrightarrow{HNO_3} \underset{\underline{2}}{O_2NO\text{-}(CH_2CH_2O)_n\ CH_2CH_2ONO_2}$$

where n is an integer ranging from 1 to 10. Such nitrato compounds have proven to be useful energetic compositions, such as plasticizers, for propellants and explosives. An example is triethylene glycol dinitrate (n = 2).

The conversion of such polyalkaline ether dinitrates to the corresponding diazides (3) has been reported in Wilson, E. R. and Frankel, M. B., J.Chem.Eng. Data 1982, 27, 472:

$$\underset{\underline{2}}{O_2NO\text{-}(CH_2CH_2O)_nCH_2CH_2ONO_2} \xrightarrow{NaN_3} \underset{\underline{3}}{N_3\text{-}(CH_2CH_2O)_nCH_2CH_2N_3}$$

Such compounds are also useful energetic plasticizers.

## Summary of the Invention

There is provided by the present invention a process for producing a glycidyl azide polymer azide having the general formula:

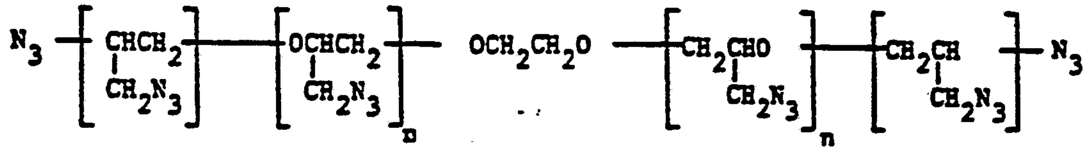

wherein n is an integer from 1 to 10.

Objects of the Invention

Therefore, it is an object of the present invention to provide improved materials useful in formulating solid propellants, explosives, and the like.

Another object of the present invention is to provide new compositions of matter.

An additional object of the present invention is to provide an energetic azide-terminated azido plasticizer.

Still another object of the present invention is to provide a new process for producing new compositions of matter.

Description of the Preferred Embodiment

In accordance with the present invention there is provided a glycidyl azide polymer azide having the following structural formula:

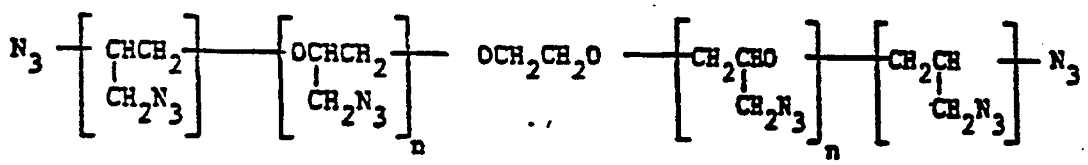

and a polyepichlorohydrin-nitrate having the following structural formula:

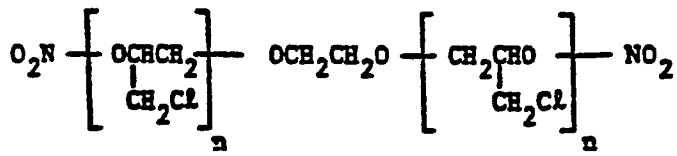

wherein n is an integer from 1 to 10.

By way of example and not limitation, the compounds of the present invention are prepared as follows:

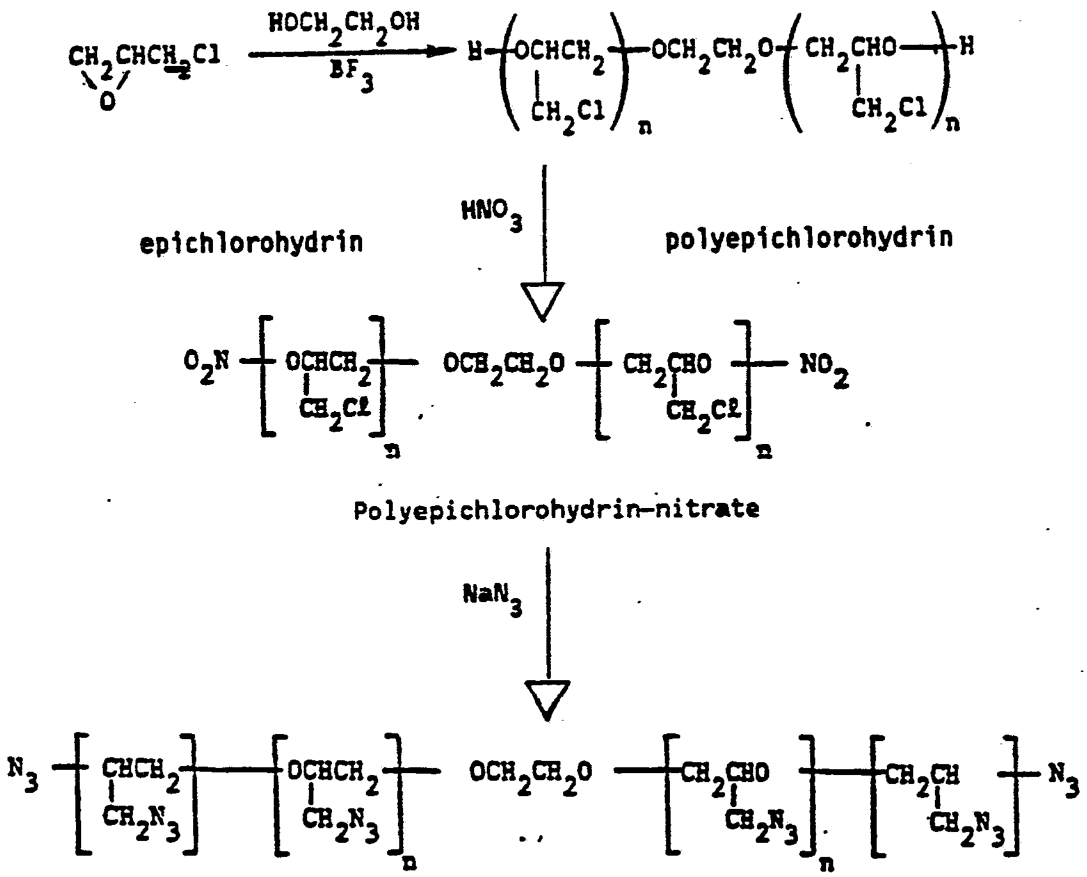


epichlorohydrin

polyepichlorohydrin

Polyepichlorohydrin-nitrate

Glycidyl Azide Polymer Azide

wherein n is an integer from about 1 to about 10.

In a suitable reaction vessel, a solution of 19.3 grams (0.3 moles) of 98% nitric acid, 20.4 grams (0.2 moles) of 96% sulfuric acid, and 51 milliters of methylene chloride was cooled to about 5° C and a solution of 47.8 grams (0.1 moles) of polyepichlorohydrin (MW 478) and 50 milliters of methylene chloride was added dropwise while maintaining the temperature at about 5° C. The polyepichlorohydrin was prepared by the polymerization of epichlorohydrin using an initiator such as ethylene glycol, propylene glycol or chloro-1,2-propanediol and a Lewis acid catalyst such as boron trifluoride.

This reaction mixture was stirred for an additional hour at about 5° C and quenched on ice. The methylene chloride solution was separated, washed first with water and then with 5% sodium bicarbonate solution. The methylene chloride solution was then again washed with water and dried over anhydrous sodium sulfate and concentrated to provide 50.7 grams (89.3%) of polyepichlorohydrin-nitrate.

The polyepichlorohydrin-nitrate was next combined with 50 milliters of dimethylsulfoxide *, and 42.2 grams (0.65 moles) of sodium azide. This mixture was heated for about 8 hours at about 100° C, cooled, and quenched on water. The solution was extracted with 150 milliters of methylene chloride. The organic solution was next washed with 700 milliters of water 5 times and subsequently dried over anhydrous magnesium sulfate and then passed through a silica gel column. Concentration of the final solution gave 23.1 grams (43.8%) of glycidyl/ azide polymer azide. The product was characterized and identified as set forth in Table 1.

*NOTE: While dimethylsulfoxide is preferred, dimethylformamide may also be used as the organic solvent.

## Table 1

| NAME: | GLYCIDYL AZIDE POLYMER AZIDE | | |
|---|---|---|---|
| STRUCTURE: | 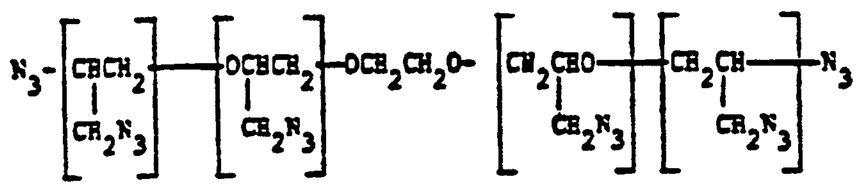 | | |
| FORMULA: | $C_{14}H_{24}N_{18}O_4$ | | |
| MOLECULAR WEIGHT: | 508 | | |
| ELEMENTAL ANALYSES: | C | H | N |
| Calculated: | 33.07 | 4.72 | 49.61 |
| Found: | 33.21 | 4.93 | 49.33 |
| INFRARED SPECTRUM: | $N_3$(4.7µ) | | |
| APPEARANCE: | Light Yellow Liquid | | |
| REFRACTIVE INDEX: | 1.5108 @ 28°C | | |
| DENSITY: | 1.24 g/cc | | |
| FREEZING POINT: | -67°C | | |
| DSC: | 187°C (Onset of Exotherm) | | |
| WEIGHT LOSS: | 0.35% after 140 hrs at 74°C | | |
| IMPACT SENSITIVITY: | 144 in-lb | | |
| $\Delta H_f$: | +273 Kcal/mole | | |

Obviously, numerous variations and modifications may be made without departing from the present invention. Accordingly, it should be clearly understood that the form of the present invention described above is illustrative only and is not intended to limit the scope of the present invention.

What is claimed and desired to be secured by Letters Patent of the United States is:

## Claims

1. A process for producing a glycidyl azide polymer azide of the formula:

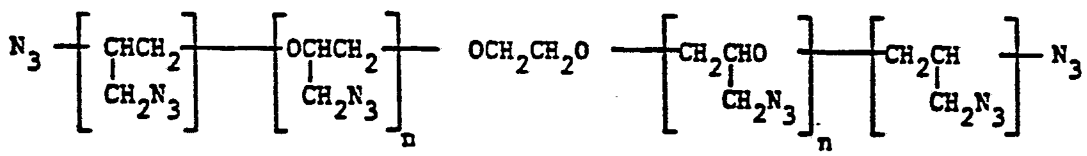

**by reacting polyepichlorohydrin-nitrate of the formula:**

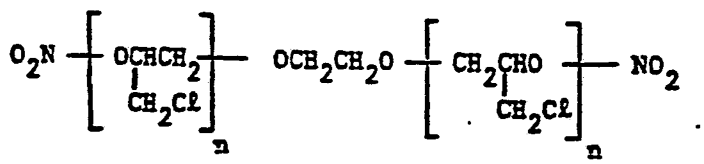

with sodium azide in a polar organic solvent wherein n is an integer from 1 to 10.

2. A process for producing a glycidyl azide polymer azide according to Claim 1 in which the organic solvent is selected from the group consisting of dimethylformamide or dimethylsulfoxide.

3. A process for producing polyepichlorohydrin-nitrate of the formula:

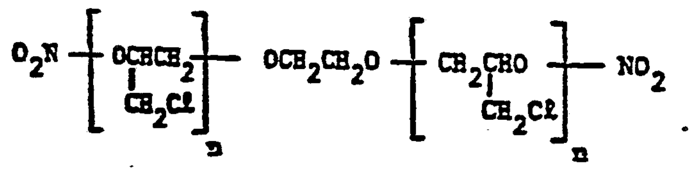

by reacting $HNO_3$ with polyepichlorohydrin of the formula:

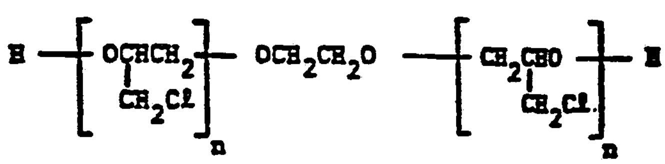

wherein n is an integer of from about 1 to 10.

4. A glycidyl azide polymer azide produced according to the process of Claim 1 having the formula:

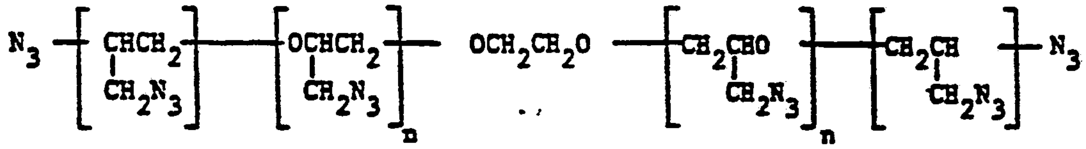

wherein n is an integer from 1 to 10.

5. As a composition of matter, polyepichlorohydrin-nitrate having the general formula as in Claim 3.